Europäisches Patentamt

European Patent Office

Office européen des brevets

(19)

(11) Publication number: **0 208 395**
**B1**

# EUROPEAN PATENT SPECIFICATION

(12)

(45) Date of publication of the patent specification:
20.12.89

(51) Int. Cl.⁴: **A61M 35/00**

(21) Application number: 86303220.7

(22) Date of filing: 29.04.86

(54) Transdermal delivery system.

(30) Priority: 16.05.85 GB 8512358

(43) Date of publication of application:
14.01.87 Bulletin 87/3

(45) Publication of the grant of the patent:
20.12.89 Bulletin 89/51

(84) Designated Contracting States:
AT BE CH DE FR GB IT LI LU NL SE

(56) References cited:
EP-A- 0 114 125
EP-A- 0 171 800
DE-A- 2 902 183
FR-A- 2 562 800
GB-A- 845 841
GB-A- 2 093 694
GB-A- 2 100 605
US-A- 4 486 193

(73) Proprietor: Euroceltique SA, 122 Boulevard de la
Petrusse, Luxembourg(LU)

(72) Inventor: Jenkins, Anthony William, Bridge Farm West
Street, Comberton Cambridge(GB)
Inventor: Leslie, Stewart Thomas, 4 Babraham Road,
Cambridge CB2 2RA(GB)
Inventor: Miller, Ronald Brown, Bruderholzallee 191,
CH-4059 Basel(CH)

(74) Representative: James, Stephen Richard,, Napp
Pharmaceutical Group Cambridge Science Park Milton
Road, Cambridge CB4 4GW(GB)

## Description

The present invention relates to a transdermal delivery system, in particular to a transdermal delivery system for the administration of a drug to the skin in a controlled manner. The invention also relates to a method for preparing the present transdermal delivery system.

Drug delivery systems for transdermal drug administration are known in the prior art. For example, GB-A 2 081 582 describes a transdermal nitroglycerin pad. This pad consists of a backing, which is impervious to nitroglycerin absorption and transport, and, affixed to the backing, a silicone matrix. The matrix is prepared by cross linking silicone rubber in the presence of a mixture of a hydrophilic solvent (polyethylene glycol containing nitroglycerin) and a hydrophobic solvent (mineral oil or a triglyceride of a saturated coconut oil acid). The presence of the hydrophobic solvent is said to enhance nitroglycerin dispersion and transport.

A problem associated with this pad is that, in order to accommodate an amount of nitroglycerin that is appropriate to transdermal administration, the pad will tend to be rather bulky.

GB-A 2 100 605, on the other hand, relates to a medical bandage for transdermal drug delivery. The bandage consists of a backing, a reservoir containing the drug, a rate controlling membrane and an adhesive layer. The reservoir is generally a mixture of a naturally occurring or synthetic oil, a rheological agent such as cellulosic, polysaccharide or silicon agents, and the drug. The membrane, which is adjacent to the reservoir, is microporous. It is this property of the membrane that controls the rate of drug release from the reservoir, since the rate of release is determined by the rate of passage of the drug through the body of the membrane.

One problem of this type of system is that the choice of membranes to control the rate of drug release is limited, making the system fairly inflexible.

Another problem associated with this type of bandage is that seepage of the drug vehicle occurs along the adhesive layer, which may lead to a loss of skin adhesion.

It is an object of the present invention to provide transdermal delivery systems that overcome one or more of these problems.

It is a further object of the present invention to provide a compact transdermal delivery system having a macroporous, rather than a microporous, layer through which the drug passes.

It is still further object of the present invention to provide a compact transdermal delivery system in which the migration of the drug along the adhesive layer is prevented.

Yet further objects and advantages of the present invention will become apparent from the following detailed description thereof.

According to a first aspect of the present invention, therefore, there is provided a transdermal delivery system for the transdermal administration of a drug comprising

(a) a backing that is substantially impermeable to the drug,

(b) a matrix, adjacent to a surface of the backing, the matrix comprising a drug containing gel comprising a fluid, a fluid gelling agent and the drug, and

(c) a perforated sheet, adjacent to the matrix, that allows passage of the drug containing gel through its perforations.

(d) in contact with the rate controlling sheet, a contact adhesive layer, and

(e) in contact with the adhesive layer, a protecting layer, characterised in that the perforated sheet has a thickness between 0.1 and 0.4 mm and in that the matrix is surrounded by a channel, formed in the rate controlling sheet and the contact adhesive layer, which inhibits the lateral movement of the drug containing gel.

The fluid may be any fluid that is compatible with the drug and in which the drug is soluble enough to give a drug concentration in the fluid of at least 0.1% (w/w) at 30⊠C. In order to allow effective transdermal delivery of the drug, however, the drug should not be very soluble in the fluid. Preferably, the maximum concentration, of the drug in a given fluid, that may be achieved, at 30⊠C, should be 10% (w/w). Most preferably, the fluid is chosen such that, when in the transdermal delivery system of this invention, the concentration of a unit dose of the drug in the fluid is above 75%, especially above 85%, of the saturated concentration (of the drug in the fluid) at 30⊠C.

Suitable fluids include water, propylene glycol, polyethylene glycol and propylene carbonate. Other suitable, biologically acceptable fluids will, however, be well known to those skilled in the art.

The fluid gelling agent may be any thixotropic and/or thickening agent that forms a gel with the chosen fluid and that is compatible with the drug. The agent is used to reduce the ability of the fluid to flow which results in an increased viscosity (of the fluid) and eventually in the formation of a gel. The agent essentially eliminates slump (or run) in the matrix and leads to the controlled release of the drug from the gel. The agent also serves to prevent settling of the drug during storage.

Suitable fluid gelling agents include:

(a) Water-soluble cellulosic materials, such as carboxyalkylcelluloses and alkali metal derivatives of carboxyalkylcelluloses. Preferably, however, the cellulosic material is a water-soluble hydroxyalkylcellulose, particularly a $C_1$-$C_4$ hydroxyalkyl cellulose. The present inventors have found that the hydroxyethyl cellulose sold by Hercules Powder Company under the Trade Mark Natrosol 250 is especially suitable for use in the present gel.

The amount of cellulosic material employed in the gel of the present delivery system is determined by the rate of (and efficiency of) drug release that is required. Preferably the amount of cellulosic material used will be between 1% and 6%, especially between 2.5% and 3.5%, by weight of the total gel.

Although, in preferred embodiments of the present transdermal system, the cellulosic material in the gel is hydrated by water only, minor quantities of polar, biologically acceptable solvents (especially alcohols) may be present in the hydrating medium without detriment to the delivery system.

(b) Water-soluble polysaccharides, such as agar, agarose, algin, sodium alginate, potassium alginate, carrageenan, kappa-carrageenan, lambda-carrageenan, fucoidan, furcellaran, laminaron, gum tragacanth, hypnea, eucheuma, gum arabic, gum ghatti, gam karaya, guar gum, locust bean gum, quince psyllium, okra gum, arabinogalactin, perctin, xanthan, scleroglucan, dextran, amylose, amylosepectin, dextrin and the like.

(c) Silicon containing materials, such as fumed silica, reagent grade sand, precipitated silica, amorphous silica, colloidal silicon dioxide, fused silica, silica gel, quartz and particulate siliceous materials commercially available (e.g. Syloid (Trade Mark), Cabosil (Trade Mark), Aerosil (Trade Mark) and Whitelite (Trade Mark)).
The amount of silicon material used in the present gel is preferably between 0.1% and 25%, especially between 1% and 15%, by weight of the total gel.

(d) Long chain ($C_8-C_{36}$) fatty alcohols, such as cetyl alcohol, stearyl alcohol and cetostearyl alcohol.

The gel may also contain minor quantities of a suitable preservative, for example Octhilinone (Kathon, Trade Mark).

Any drug that is compatible with the fluid and the fluid gelling agent and that can be administered percutaneously through the skin for passage into the systemic circulation may be incorporated into the present delivery system.

Examples include:

(a) Vasodilators, such as glyceryl nitrate, amyl nitrate, isosorbide mono- and dinitrate, triethanolamine trinitrate, nifedipine, verapamil, nicotinic acid,

(b) Antihypertensives, such as clonidine, propranolol,

(c) Diuretics, such as hydrochlorothiazide, bendrofluazide, ethacrynic acid,

(d) Antihistamines, such as 2-diphenylmethoxy-N, N-dimethylethanamine, clemastine fumarate, mepyramine,

(e) Anticholinergics, such as scopolamine, atropine, methscopolamine bromide,

(f) Analgesics, such as codeine, morphine, aspirin, sodium salicylate, salicylamide, hydromorphone, phenazocine

(g) Bronchodilators, such as salbutamol, alpha-(1-(methylamino) ethyl)-benzenemethanol, isoproterenol,

(h) Steroids, such as 6-methylprednisolone, methyltestosterone, fluoxymesterone, estrone, estradiol, ethinylestradiol, 17-hydroxyprogesterone acetate, medroxyprogesterone acetate, 19-norprogesterone, norethindrone, hydrocortisone, dexamethasone, prednisolone,

(i) Non-steroidal hormones, such as thyroxine, vasopressin, heparin, insulin,

(j) Antidiabetics, such as chlorpropamide, glibenclamide,

(k) Antimalarials, such as 4-aminoquinolines, 9-aminoquinolines, pyrimethamine,

(l) Vitamins, Essential Amino Acids and Essential Fats,

(m) Sedatives and Hypnotics, such as pentabarbital sodium, phenobarbital, sodium phenobarbital, secobarbital sodium, carbromal,

(n) Psychic energisers, such as 3-(2-aminopropyl)indole acetate and 3-(2-aminobutyl)indole acetate,

(o) Tranquilisers, such as reserpine, chlorpromazine hydrochloride, thiopropazate hydrochloride,

(p) Antimicrobials, such as penicillins, tetracyclines, oxytetracyclines, chlortetracyclines, chloramphenicol, sulphonamides,

(q) Antiinflammatorys, such as indomethacin and ibuprofen,

Of the above materials, nitroglycerine, nifedipine, salbutamol and hydromorphone are particularly preferred for use in the present delivery system.

It will be appreciated that the drug may be added to the above matrix not only in the form of the pure chemical compound, but also in admixture with other drugs which may be transdermally applied or with other ingredients which are not incompatible with the desired objective of transdermally administering the drug to a patient. Thus, simple pharmacologically acceptable derivatives of the drugs, such as ethers, esters, amides, acetals, salts and the like may be used. In some cases such derivatives may actually be preferred.

The amount of drug in the gel will generally be enough to maintain a therapeutic level of the drug in the bloodstream for a predetermined period. The precise amount will depend, inter alia, on the unit dose of the drug usually administered per day and the number of applications of the present transdermal delivery system envisaged per day. The drug concentration is preferably between 0.1% and 20.0%, most preferably between 0.5% and 15.0% by weight of the total gel.

The total weight of gel in the present delivery system will preferably be between 0.2gm and 10gm, especially between 0.5gm and 5gm.

The perforated sheet is a perforated sheet of a polymeric material. Preferably the type of polymeric material and the thickness of the sheet are chosen such that the rate of diffusion of the drug through the sheet (other than through the perforations) is low, usually below about $15\mu g\ hr^{-1}cm^{-2}$. Suitable materials include polyolefins such as polyethylene, polypropylene, ethylene-propylene copolymers, polyisoprene, polyamides, polyesters such as ethacrylates, terephthalates, ethylene-vinyl acetate copolymer, ethylene-vinyl lower alkyl acetate copolymers, and polyacrylonitrile. Preferably however, the perforated sheet is prepared from an ethylene/vinyl acetate copolymer and has a thickness between 0.1 and 0.4mm, most preferably between 0.15 and 0.2mm. Preferably the ethylene/vinyl acetate copolymer sheet is that sold by the 3M Company as 3M Brand surgical tape, No. 1527L (Trade Mark).

The area of the release surface will be between the normal limits associated with transdermal systems of the present type. Generally an area between 5 and 20 cm² is employed. However, for high doses a larger area may be employed.

The area of the release surface, together with the size and number of perforations, the quantity of gel, the concentration of the drug in the gel and other variables, determine the duration of therapeutic action of the present delivery system. Although delivery systems having very short or very long duration times may be prepared, they will generally have a duration time of between 8 and 120 hr, especially between 12 and 72 hr.

It will be seen that the present delivery system differs fundamentally from the system described in GB-A 2 100 605. In that prior art system, the primary release of nitroglycerin is by the diffusion of nitroglycerin alone (or in conjunction with an organic oil) through the pores of a microporous membrane. In the present case, by contrast, the primary release of the drug is by the movement of the drug containing gel through the perforations in the polymeric sheet and by diffusion of the drug through the gel.

Since the choice of materials to produce the gel is far greater than the choice of polymers to form a rate controlling sheet, the present system has a greater flexibility for drug release (choice of drug, rate of drug release, etc.) than the system of GB-A 2 100 605.

The backing to the present delivery system must be substantially impermeable to the drug. Suitable materials or combinations of materials will be well known to those skilled in this art. Thus the backing may be prepared from organic polymers such as nylon and polyethylene terephthalate. Alternatively the backing may be a metal foil such as aluminium foil or a laminate of such a foil and an organic polymer. Further variations may readily be envisaged. Some are described in GB-A 2 100 605, the contents of which disclosure are hereby incorporated herein by way of reference.

In order to facilitate the use of the present delivery system, the system, in another aspect of the present invention, further comprises a contact adhesive layer and a protecting layer. The adhesive layer, which is in contact with the perforated sheet, may be prepared using any of the biologically compatible, contact adhesives that are known in this art. These include cyanoacrylates, acrylates, colloidal gums, polyisobutylene adhesives, silicone medical adhesives and the like. In one preferred embodiment of the present system the adhesive layer initially contains a relatively high concentration of the drug. This ensures a high initial delivery of the drug to the patient. The protecting layer, which is in contact with the adhesive layer and is removed prior to use may be prepared, for example, from known types of protective paper, metal foil or polymer. Suitable materials will be well known to those skilled in this art. The protecting layer must be substantially impermeable to the drug and the drug containing gel. It must also be easily removed from the present delivery system prior to use.

The present inventors have found that in transdermal delivery systems in which an adhesive layer is sealed to a backing to enclose a matrix, there may be migration of the matrix's contents. This may result in the lowering of skin adhesion or, in the case of a volatile drug, loss of the matrix's active constituent.

In two further aspects of the present invention therefore delivery systems are provided which overcome these problems. In the first, the backing and the rate controlling sheet/adhesive layer lamina are joined by an outer and an inner seal. Between these two seals the rate controlling sheet/adhesive layer lamina is absent. In this embodiment therefore the matrix is surrounded by a channel through which the matrix's contents may not readily pass i.e. which inhibits the lateral movement of the drug containing gel. In this way the outer (circumferential) portion of the rate controlling sheet/adhesive layer lamina is kept free from the matrix's contents. This in turn ensures that the skin adhesion of this outer portion (at least) remains high.

In the second aspect, the loss of a volatile constituent from a delivery system having a channel surrounding the matrix (as described above) is prevented by forming a surrounding (circumferential) seal, between the backing and the protecting layer, in this matrix surrounding channel.

In order to facilitate the manufacture of the present transdermal delivery system there is also provided, in a further aspect of the present invention, a process for the preparation of a transdermal delivery system comprising mixing a fluid, a fluid gelling agent and a drug to form a drug containing gel.

The drug containing gel may then be incorporated in the present delivery system by depositing the gel on a perforated sheet, optionally, having attached thereto an adhesive layer and a protecting layer. The system may be completed in this case by heat sealing a backing (over the gel) to the perforated sheet.

Alternatively the gel may be deposited on the backing, in which case the perforated sheet may be heat sealed (over the gel) to the backing. In the case of perforated sheet delivery systems having no surrounding channel (in the perforated sheet/adhesive layer lamina), the system may be prepared on a conventional sachet machine. This represents a major advantage of the present invention.

The present transdermal delivery system and processes for its preparations will now be described by way of example only, with particular reference to the Figures in which:

Figure 1 is a cross-sectional view of a transdermal delivery system according to the present invention,

Figure 2 is a cross-sectional view of a transdermal delivery system according to this invention in which the matrix is surrounded by a channel,

Figure 3 is a cross-sectional view of a transdermal delivery system according to this invention in which the backing and the protecting layer are sealed in the matrix surrounding channel, and

Figure 4 is the nitroglycerin plasma level achieved (as a function of time) by six patients using a delivery system according to the present invention.

Example 1

Gel Preparation

0.7g of Nitroglycerin (in the form of 10% TNG on lactose (BP)) and 0.1g of Octhilinone (Kathon CG, Trade Mark) were dissolved in distilled water (89.9g), by magnetic stirring in a covered vessel warmed to a temperature between 25⊠ and 45⊠C.

When a clear solution was formed, hydroxyethyl cellulose (Natrosol 250 HHBR, Trade Mark, 3g) was added slowly with stirring. After the addition of the hydroxyethyl cellulose was completed, stirring was continued for a further 5 mins until a cohesive gel had formed.

Delivery System Preparation

An aluminium backing (3M 1006, Trade Mark) was heat sealed to a lined, self adhesive ethylene/vinyl acetate copolymer (EVA) macropore membrane (3M 1527L, Trade Mark) to form a 10 cm² elliptical delivery system with one end open. 2.3g of the above gel was then injected into the system's matrix via a tube attached to a syringe. The ellipse was then closed by further heat sealing, and the delivery system was cut to shape, allowing a protruding tab of the release liner.

The delivery system was stored in a heat sealed aluminium foil envelope.

Example 2

The delivery system of Example 1 was prepared except that isosorbide dinitrate on lactose replaced the TNG on lactose.

Example 3

The delivery system of Example 1 was prepared except that octyl nitrate (0.7g) replaced the TNG on lactose, the gel being made up to 100gm in weight oy the addition of distilled water.

Example 4

The delivery system of Example 1 was prepared except that isosorbide mononitrate replaced the TNG on lactose.

Example 5

Gel Preparation

Salbutamol (2g) was dissolved in propylene glycol (84.2g) by stirring and gentle warming. Cetostearyl alcohol (13.8g) was then added and the mixture was heated to 60⊠C, with stirring. The mixture was then allowed to cool to room temperature.

Delivery System Preparation

The delivery system was prepared as described in Example 1, except that 1.5g of the above salbutamol gel was injected into the matrix.

Example 6

Gel Preparation

Nifedipine (1.5g) was dissolved in polyethylene glycol 200 (88.5g) by stirring and gentle warming. Fumed silica (10.0g) was then added, with stirring.

Delivery System Preparation

The delivery system was prepared as described in Example 1, except that 1.33g of the above nifedipine gel was injected into the matrix.

Example 7

Gel Preparation

Propylene carbonate (89.3g) was stirred into the fumed silica (10.2g) at 20⊠C. Hydromorphone (0.5g) was then dissolved in the mixture, with stirring and gentle warming. The mixture was then allowed to cool.

Delivery System Preparation

The delivery system was prepared as described in Example 1, except that 2g of the above hydromorphone gel was injected into the matrix.

Example 8

Gel Preparation

Cetostearyl alcohol (18g), sodium alkylsulphate (2g), oleic acid decyl ester (10g), sorbitan monooleate (1.4g), polyoxyethylene monooleate (3.6g), Nipastat (Trade Mark, 2g) and propylene glycol (20g) were mixed together at 80⊠C with stirring, until a homogeneous mixture was obtained. Aspirin (2g) and lactose (1g) were then stirred into the mixture. Hot aqueous ethanol (43g), was then added and the mixture was cooled, with stirring, to produce a gel.

Delivery System Preparation

The delivery system was prepared as described in Example 1, except that 2g of the above aspirin gel was injected into the matrix.

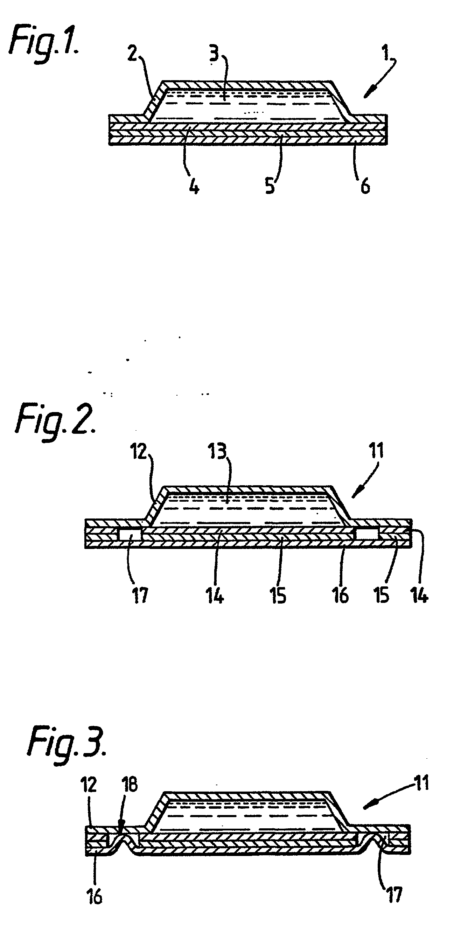

Referring to the Figures, Figure 1 illustrates an elliptically shaped transdermal delivery system (1) which has a backing (2) of occlusive clear plastic or aluminised heat sealable plastic. 2.3gm of the nitroglycerin containing gel of Example 1 (3) is sealed in the backing (2) by a perforated ethylene/vinyl acetate copolymer sheet (4). Incorporated in this sheet is an adhesive layer (5) and protecting paper layer (6).

Figure 2 illustrates another transdermal delivery system (11) in which the perforated sheet (14) and the adhesive layer (15) are broken to form a matrix surrounding channel (17).

This embodiment of the present delivery system prevents migration of the gel (13) along the adhesive layer (15), thereby enhancing skin adhesion.

Figure 3 illustrates a delivery system of the type illustrated in Figure 2 but with an additional, weak circumferential seal (18), between an aluminium backing (12) and an aluminium protecting layer (16), in the matrix surrounding channel (17).

This embodiment of the present delivery system may be used with volatile drugs to prevent loss of such drugs from the unopened delivery system.

Clinical Trial

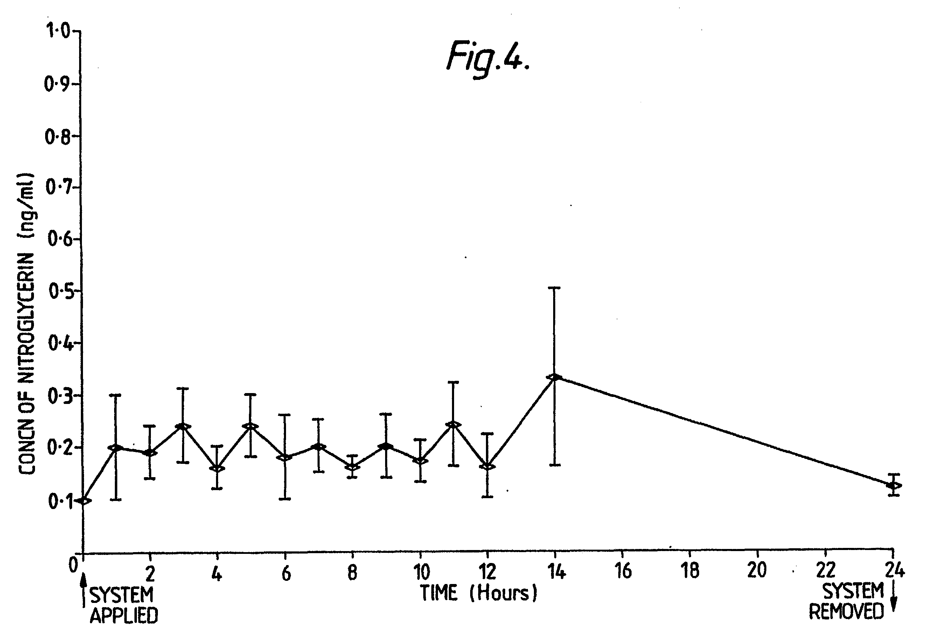

The delivery of nitroglycerin from delivery systems prepared as in Example 1 above was then demonstrated in the following study. Two delivery systems according to Example 1, each containing 16mg. of nitroglycerin, were placed on the chests of six volunteer subjects. Blood samples were taken on the hour for 12 hours and then after 14 and 24 hours. The samples were analysed for nitroglycerin using HPLC followed by thermal energy analyser detection. Figure 4 depicts the nitroglycerin plasma concentration as a function of time for the six patients.

**Claims**

1. A transdermal delivery system for the transdermal administration of a drug comprising
   (a) a backing that is substantially impermeable to the drug,
   (b) a matrix, adjacent to a surface of the backing, the matrix comprising a drug containing gel comprising a fluid, a fluid gelling agent and the drug, and
   (c) a sheet, adjacent to the matrix, that controls the rate of gel release from the system,
   (d) in contact with the rate controlling sheet, a contact adhesive layer (15), and
   (e) in contact with the adhesive layer, a protecting layer (16),
   characterised in that the perforated sheet has a thickness between 0.1 and 0.4 mm and in that the matrix is surrounded by a channel (17), formed in the rate controlling sheet and the contact adhesive layer, which inhibits the lateral movement of the drug containing gel.

2. A transdermal delivery system according to claim 1 characterised in that the rate of diffusion of the drug through the sheet (other than through the perforations) is below about 150µghr$^{-1}$cm$^{-2}$.

3. A transdermal delivery system according to either claim 1 or claim 2 characterised in that the perforated sheet comprises an ethylene-vinyl acetate copolymer.

4. A transdermal delivery system according to claim 3 characterised in that the perforated sheet has a thickness between 0.1 and 0.4mm.

**Patentansprüche**

1. Transdermales Abgabesystem (11) zur transdermalen Verabreichung eines Arzneimittels, bestehend aus
   (a) einer für das Arzneimittel weitgehend undurchlässigen Rückenplatte (12),
   (b) einer an die Oberfläche der Rückenplatte angrenzenden Matrix (13), wobei die Matrix ein arzneimittelenthaltendes Gel mit einer Flüssigkeit, einem die Flüssigkeit gelierenden Mittel und dem Arzneimittel umfaßt,
   (c) einer an die Matrix angrenzenden durchlochten Folie (14), welche die Freisetzungsgeschwindigkeit des Arzneimittels oder Gels aus dem System kontrolliert,
   (d) einer mit der geschwindigkeitskontrollierenden Folie in Kontakt stehenden Haftklebeschicht (15) und
   (e) einer mit der Klebeschicht in Kontakt stehenden Schutzschicht (16),
   dadurch gekennzeichnet, daß die durchlochte Folie eine Dicke zwischen 0,1 und 0,4 mm aufweist und die Matrix von einem, in der geschwindigkeitskontrollierenden Folie und der Haftklebeschicht gebildeten Kanal (17) umgeben ist, der die seitliche Bewegung des arzneimittelenthaltenden Gels hemmt.

2. Transdermales Abgabesystem nach Anspruch 1, worin die Matrix in dem die Matrix umgebenden Kanal zwischen der Rückenplatte und der Schutzschicht von einer Dichtung (18) umgeben ist.

3. Transdermales Abgabesystem nach Anspruch 1 oder 2, worin die Diffusionsgeschwindigkeit des Arzneimittels durch die Folie (abgesehen von den Durchlochungen) unter ungefähr 150 µm hr$^{-1}$cm$^{-2}$ liegt.

4. Transdermales Abgabesystem nach einem der Ansprüche 1 bis 3, worin die durchlochte Folie aus einem Äthylen/Vinylacetat-Copolymer besteht.

**Revendications**

1. Un système d'application transdermique (11) en vue de l'administration transdermique d'un médicament comprenant
   a) un support (12) sensiblement imperméable au médicament,
   b) une matrice (13), adjacente à une face du support, la matrice comprenant un gel contenant un médicament comprenant un fluide, un agent de gélification du fluide et le médicament.
   c) une feuille perforée (14), adjacente à la matrice, qui contrôle le débit de libération du médicament ou du gel à partir du système,
   d) en contact avec la feuille de contrôle du débit, une couche adhésive de contact (15), et
   e) en contact avec la couche adhésive, une couche de protection (16),
   caractérisé en ce que la feuille perforée a une épaisseur comprise entre 0,1 et 0,4 mm et en ce que la matrice est entourée d'un canal (17) formé dans la feuille de contrôle du débit et la couche adhésive de contact, qui empêche le mouvement latéral du gel contenant le médicament.

2. Un système d'application transdermique selon la revendication 1, dans lequel la matrice est entourée par un joint (18), entre le supoort et la couche de protection, dans le canal entourant la matrice.

3. Un système d'application transdermique selon l'une quelconque des revendications 1 ou 2, dans lequel le débit de diffusion du médicament à travers la feuille (distinct de celui à travers les perforations) est inférieur à environ 150 µm heure$^{-1}$ cm$^2$.

4. Un système d'application transdermique selon l'une quelconque des revendications 1 à 3, dans lequel la feuille perforée comprend un copolymère d'éthylène/acétate de vinyle.

*Fig.1.*

*Fig.2.*

*Fig.3.*

*Fig.4.*

EP 0 208 395 B1